# EUROPEAN PATENT SPECIFICATION

(11) **EP 2 011 497 B1**
(45) Date of publication and mention of the grant of the patent: **04.05.2011**
(21) Application number: 07747831.1
(22) Date of filing: 27.02.2007
(51) Int. Cl.: A61K 31/4015, A61P 9/10, A61P 25/00, A61P 25/28

(54) **Phenotropil for the prophylaxis and treatment of hemorrhagic stroke and acute phase of ischemic stroke**
Phenotropil zur Prophylaxe und Behandlung von hämorrhagischem Schlaganfall und der akuten Phase eines ischämischen Schlaganfalls
Phenotropil pour la prophylaxie et le traitement de l' AVC hémorragique et de la phase aiguë de l'AVC ischémique

(30) Priority: 28.03.2006 RU 2006109678
(43) Date of publication of application: 07.01.2009
(73) Proprietor: Akhapkina, Valentina Ivanovna, 105264 Moscow (RU)
(72) Inventor: VORONINA, Tatiana Aleksandrovna, Moscow, 119048 (RU); AKHAPKIN, Roman Vitalevich, Moscow, 105264 (RU)
(74) Representative: Glawe, Delfs, Moll
(86) International application number: PCT/RU2007/000094
(87) International publication number: WO 2007/111528

(56) References cited:
- RU-C1- 2 050 851
- RU-C1- 2 050 851
- RU-C2- 2 192 585
- NV SELYANINA, AA SHUTOV: "Experience in applying phenotropyl in patients with initial manifestations of insufficient blood circulation in the brain" NERVOUS DISEASES, [Online] no. 4, 2005, XP002523606 Retrieved from the Internet: URL:http://www.medi.ru/doc/310118.htm> [retrieved on 2009-04-14]
- SS KORSAKOVA: "Clinical and immunological effects of phenotropyl on the effects of cerebral stroke" JOURNAL OF NEUROLOGY AND PSYCHIATRY, [Online] no. 5, 2005, XP003018141 Retrieved from the Internet: URL:http://www.medi.ru/doc/310113.htm> [retrieved on 2009-04-14]
- VI AHAPKINA, TA VORONINA: "The spectrum of pharmacological effects of phenotropyl" FARMATEKA, [Online] no. 13, 2005, XP003018142 Retrieved from the Internet: URL:http://www.medi.ru/doc/310115.htm> [retrieved on 2009-04-14]
- SELIYANINA N.V. ET AL.: 'Opyt primenenya fenotropila bolnykh s nachalnymi proyavlenyami nedostotochnosti krovosnabzhenya mozga' NERVNYE BOLEZNI, [Online] no. 4, 2005, XP003018140 Retrieved from the Internet: <URL:http://www.medi.ru/doc/310118.htm>
- GERASIMOVA M.M.: 'Kliniko-Immunologicheski aspekti vlyanya fenotropila na posledstviya tserebralnogo insulta' ZHURNAL NEBROLOGII I PSIKHYATRII IM S.S. KORSAKOVA, [Online] vol. 105, no. 5, 2005, XP003018141 Retrieved from the Internet: <URL:http://www.medi.ru/doc/310113.htm>
- AKHAPKINA V.I. ET AL.: 'Spektr farmakologicheskikh effektov fenotropila' FARMATEKA, [Online] no. 13, 2005, XP003018142 Retrieved from the Internet: <URL:http://www.medi.ru/doc/310115.htm>

## Description

### Field of the invention

This invention is defined in the claims and relates to the field of medicine, in particular to pharmacology, and relates to medicinal preparations exhibiting complex, multicomponent action on the functional state of the central nervous system (CNS) and brain vessels.

### Prior Art

A wide range of neurotropic and cerebrovascular medicinal agents relating to various pharmacological groups, which are used in complex therapy of CNS pathologies at cerebrovascular diseases among which strokes of various ethyology take lead. At this, each preparation of different pharmacological direction, which is included into a therapeutic complex, is purposefully aimed at specifically eliminating intensity of some or other symptom in symptomatic complexes of psychoneurological syndromes and diseases. Polypragmasy has a number of significant shortcomings associated both with complexity of making schemes of rational dose and schemes of simultaneous use of many medicinal agents and with forecasting their efficiency and tolerance as well as possible synergy of side effects.

### Disclosure of the Invention

The invention is defined in the claims.

The objective of this invention is to develop a universal, from the point of neuropsychopharmacology, medicinal agent.

If this objective is achieved, it will become possible to attain a technical effect consisting in the medicinal agent's capability of having, at monotherapy, a complex effect on the functional state of the CNS and brain vessels at neurocerebral and cerebrovascular diseases and possessing therapeutic and prophylactic properties preventing pathological development of CNS diseases.

The above technical effect can be achieved by the use of N-carbomoyl-methyl-4-phenyl-2-pyrrolidon (Phenotropil) as an agent exhibiting neurotropic-neuromodulator, cerebrovascular and anti-stroke activity, which is known as a antihypertensive and anti-ischemic agent (at ischemic heart disease (IHD)) (USSR Inventor's Certificate No. 797219, A61K 21/40, 3C07D 207/26; RF Patent No. 2183117, C1, A61K 31/40; Eurasian Patent No. 002380, B1, A61K 31/4015); an agent and a pharmaceutical composition that exhibit nootropic activity (RF Patent No. 2050851, C1, A61K 31/40; RF Patent No. 2240783, C1, A61K 9/20, 31/40, A61P 25/28); and as an agent exhibiting antidepressant activity (RF Patent No. 2232578, C1, A61K 31/41, 52, A61P 25/24).

The effects of this agent on an acute cerebrovascular pathology have not been described. It is proposed to use N-carbomoyl-methyl-4-phenyl-2-pyrrolidon (Phenotropil) as a neurotropic-neuromodulator, cerebrovascular and anti-stroke agent, which has a number of advantages compared to use of agents relating to various pharmacological groups, namely:
pronounced multicomponent efficiency; simplicity of dose and use in any medicinal form; relatively low toxicity (LD30⁼800 mg/Kg); lack of pronounced side effects; at course intake no addiction, dependence or tolerance is developed, the agent is not metabolized in the organism and goes out of it in the unchanged form.

As the model for assessing the proposed effects of Phenotropil the most severe and most widely spread form of cerebrovascular pathology - stroke - has been chosen, for which therapy no significant results have been obtained so far.

In developed countries brain vessel diseases rank third in the causes of death and more frequently lead to invalidity than the others. Loss of great numbers of able-bodies population requires the greatest economic expenses compared to most other diseases (see: Ye.I. Gusev, V.I. Skvortsova et al., 2003; N.V. Vereschagin, N.V. Varakin, 2001; T.R. Harrison et al., 1997). There are two main directions in the stroke course: thrombolysis, neuromodulation and neuroprotection. The thrombolysis efficiency is proven by a plurality of clinical studies; as far as medicinal agents exhibiting neuromodulator and neuroprotective activity are concerned, at present they are actively searched for.

The studies have been conducted in accordance with the Manual on experimental (preclinical) study of new pharmacological substances issued by the RF Ministry of Health in 2000. The findings of the studies have been processed statistically. Average values and standard deviations for each group have been calculated. The statistic accuracy of differences between the experimental and the control groups has been evaluated with the use of the Student t-test, the percent-square test and the Mann-Whitney test. Differences have been regarded as accurate at p <0.05. (see: V.P. Borovkov, 2001; S. Glanz, 1999).

### Example 1. Study of anti-stroke action of Phenotropil with a hemorrhagic stroke model (intracerebral post-traumatic hematoma).

Experiments have been conducted on white outbred male rats having weights from 200 to 250 grams. The rats have been held in vivarium conditions with free access to meals and water, under the natural change of days and nights. For the purpose of generating hemorrhagic stroke (HS) in rats narcotized with chloral hydrate (400 mg/Kg) stereotaxic craniotomy was conducted and then brain tissue in the *capsula interna* area was destructed with a mandarin knife, and blood (0.02 to 0.03 mL) taken under a rat tongue was injected into the place of destruction. Thus, local autohemorrhagic bilateral stroke was generated in the *capsula interna* area (diameter 2 mm, depth 3 mm) without damaging the above-located brain formations and neocortex. Falsely operated animals and animals with HS fed with a saline were used as the control animals. Intragastric administration of Phenotropil at a dose of 100 mg/Kg was performed with the use of a special tube in 5 hours after the operation and then daily for 7 days at the time of the day ± 3 minutes.

During and immediately after the operation 40% of rats died. The survived rats in first hours after HS were observed for 14 days. The Phenotropil influence was evaluated for survivability of the animals, for the neurologic deficit level on the McGrow stroke-index scale in the modification of I.V. Gannushkina (1977) and in the Rotarod test, for muscular tonus maintenance in the test of chinning up on a horizontal bar, for orientation and exploration behavior in the open-field test. Study of cognitive functions was conducted on a standard unit for conditioned reflex of passive avoidance (CRPA). A test for CRPA repetition (for maintenance of a memory trail) was conducted in 24 hours after training as well as on the 3rd, 7th and 14th days after the operation. The latent time of the first entering in a dark chamber was registered when the burrow reflex maintenance was evaluated.

On the first day after the operation neurological abnormalities (90% to 100%) on the saline background were found practically in all the animals with HS, which manifested as flabbiness, slowness of motions, weakness of limbs, while such disorders were observed in 30% to 40% of the falsely operated rats (see Table 1). In the cases of HS on the Phenotropil background neurologic deficit was observed in 40% to 50% of animals and was not practically different from the group of falsely operated rats. Neurologic disorders manifested as manege movements in a circle and limb paralyses were absent in the animals with stroke, which received Phenotropil, and in the group with HS + saline deep neurologic disorders were observed in 40%, 30% and 30% of the animals, respectively.

Thus, in the acute period of HS Phenotropil manifests apparent neurotropic and cerebrovascular activity and reduces disorders of neurologic status.

Registration of muscular tonus in rats with HS showed that on the third day after stroke weakness of muscular tonus was observed in, on an average, 40% to 50% of animals and on the seventh and fourteenth days - in 38% to 36% of animals (see Table 2). In rats received Phenotropil weakness of muscular tonus was observed in 42% to 33% of animals. On the seventh day after introduction of Phenotropil weakness of muscular tonus was observed in 25% of animals, and by the fourteenth day this figure reduced to 16% and was statistically reliable compared to such figures in animals with stroke (see Table 2).

A study of the dynamics of movement coordination disorders in rats with HS showed that on the first to third days movement coordination disorders was observed in 48% to 50% of animals, and on the seventh to fourteenth days - in 38% to 45% of the survived animals (see Table 3). Phenotropil in a dose of 100 mg/Kg reduced movement coordination disorders in rats. Apparent and statistically reliable results were obtained on the seventh to fourteenth days after stroke (see Table 3).

The burrow reflex of rodents is an inborn tendency for a restricted shadowed space. On the first day after the operation all the animals maintained the burrow reflex, but in the groups with HS and on the background of one-time administration of Phenotropil the latent time of performing the reflex increased (see Table 4).

It was found during the studies that in the control group of rats, which during the whole time of the experiment received a saline (intact animals), when the CRPA was repeated in 24 hours after training (pain stimulation in a dark section of a chamber), 80% of the animals remembered a current stroke and did not enter into a dark "dangerous" chamber for the whole time of observations. In a day after training the control intact rats and the falsely operated rats well remembered a current stroke in a dark chamber and 70% to 80% of animals did not enter that dangerous section. The other rats entered the dark section with a big latent period (see Table 5a). The rats with hemorrhagic stroke (HS) the latent time of entering the dark chamber truly reduced after one day after training. Only 25% of animals did not enter the dark chamber at all, i.e., they remembered a current stroke, and memory in 75% of the rats was violated (see Table 5a).

Phenotropil in a dose of 100 mg/Kg at one-time administration in 5 hours after the operation (repetition of the CRPA in one day after training) increased the number of animals with maintained memory to 40% (animals with HS - 25%) and increased the latent time of entering a dark dangerous section. However, this beneficial effect of the Phenotropil after one-time administration was statistically unreliable as compared to that in the intact animals, but in comparison to the HS-group it increased the latent time of entering the dark chamber by 48% and the number of animals with maintained memory after one day by 60%.

In 3 days after the operation on impairing memory in animals with HS the figures were at the same level (see Table 5a). After 3 injections of Phenotropil an increase in the latent time of entering the dark chamber was observed and the number of animals, which did not enter the dark chamber, also increased, but even these effects of Phenotropil were statistically unreliable (see Table 5a) compared to those in falsely operated, but were significantly pronounced and reliable compared to those in the HS-group with a saline.

In 7 and 14 days after training the intact animals and the falsely operated animals well remembered the negative situation and performed CRPA (see Table 5b). In comparison to them, in 7th to 14th days after HS and training the animal memory of pain stimulation in a dark chamber was reliably violated. And that memory impairment was more pronounced compared to such figures in 1 and 3 days after the operation. Thus, in 7 days only 16% of the animals remembered the negative stimulation, and the other rats entered the dark dangerous section already in 28 seconds. And in 14 days only 9% of the animals preserved the CRPA (see Table 5b).

Phenotropil, which was injected to rats in a dose of 100 mg/Kg for 7 days, reconstructed memory disorders in the post-stroke period on the 7th and the 14th days after the HS operation. A statistically reliable increase (up to 40%) in the rats, which remembered the negative situation, was observed under the influence of the agent (for the HS rats - 9%). The latent time of entering a dark dangerous chamber increased app. 3 times compared to that in HS-rats (see Table 5b).

Thus, Phenotropil after the second injection is capable of reconstructing memory violated at hemorrhagic stroke on the model of the conditioned reflex of passive avoidance.

A study of orientation and exploration behavior in the conditions of the open-field method showed that during the first day after the operation a significant (almost 2 times) reduction in the total indices of motion activity and exploration behavior was observed in the HS rats. Similar indices of the rats behavior were observed on the 14th day after HS also, though the animals were slightly more active (see Table 6). Phenotropil, when its effect was registered in 3 days after HS, increased the total indices of behavior to the behavior index levels for the intact and falsely operated animals (see Table 6). When effect of Phenotropil was registered in 7 days after injection the total index of motion behavior, as compared to that in rats of the HS group, increased 2.7 times (see Table 6). In 14 days after the operation the activating effect of Phenotropil on behavior was maintained.

It can be seen in Table 7 that during 14 days after the operation one animal died in the group of falsely operated rats. In the group with HS + a saline during the first day 23% of the animals died, and by the 14th day this coefficient reached 57%. Phenotropil in a dose of 100 mg/Kg, when injected once a day for 7 days, completely prevented death of the animals.

It has been shown in the results of the studies that, compared to falsely operated animals, the following events were observed in rats with hemorrhagic stroke (intracerebral post-traumatic hematoma): pronounced neurologic deficit, impairment of movement coordination, weakness of training processes and memory, and increase in death rate of animals. Also, deepening of pathological symptomatology was noted by the 14th day of observations.

Phenotropil, when injected to animals in a dose of 100 mg/Kg in 5 hours after the operation, and then daily for 7 days, results in a significant improvement of stroke and post-stroke disorders. This agent improves indices of neurologic deficit on the McGrow scale already in a day after stroke, and when used in a course, increases muscular tonus and improves movement coordination on the 7th and 14th days after stroke. Phenotropil, when used for the second time, reconstructs memory violated by stroke, improving repetition of the conditioned reflex of passive avoidance on the 7th and 14th days after stroke. The most pronounced effect of Phenotropil is its capability of preventing death of animals with hemorrhagic stroke completely.

Thus, Phenotropil (100 mg/Kg, internally), when injected as a course for 7 days, exhibits pronounced anti-stroke action on rats on a model of hemorrhagic stroke (intracerebral post-traumatic hematoma), which manifests itself in improving the neurological status, general behavior, cognitive functions and, the most important, prevention of animal deaths.

### Example 2. Evaluation of Phenotropil efficiency on a model of acute ischemic stroke

For this study a model of local cerebral ischemia was used for rats with distal occlusion of medial cerebral artery (OMCA), which was first described by S.T. Chen (1989). The heads of animals, which were narcotized with chloral hydrate (300 mg/Kg, intraperitoneally), were rigidly fixed in a lateral position, left side upwards. After cutting the skin in the middle between the left auricle and the left eye temporal muscle fibers were moved apart up to the skull surface. Using a dental drilling machine with a bur 0.5 mm in diameter, a hole app. 3-4 mm in diameter was made in the area of the suture between the squamosal bone and the frontal bone, thus exposing the place where the medial cerebral artery and the inferior cerebral vein cross.

With the use of a microscope (Olympus SZ-CTV), under a big magnification (14.0x3.3), a special metal hook was placed under the left medial cerebral artery. Occlusion of the medial cerebral artery was performed by a method of electro-coagulation proximally the place of its bifurcation into the frontal and the parietal branches. At this time in the visual field of the microscope stoppage of the blood flow along the medial cerebral artery upstream the place of occlusion could be observed. After the operation the wound was closed layer-by-layer, and ipsilateral occlusion of the common carotid artery was performed.

For the purpose of evaluating the agent's influence on the area of ischemia the method of histochemical staining of the cerebral tissue with 2,3,5-triphenyltetrazolium chloride (TTC) was used (Bederson J.B. et al., 1986).

For this, in 72 hours after OMCA the narcotized animals were decapitated, their brain was removed from the brainpan.

Six frontal slices with thickness of 1 mm were prepared, which then were stained with TTC. Then, each stained slice was scanned on two sides and its computer planimetry was conducted with the use of MOCHA software (Jandel Scientific, version 1.2.0.0). The area of the ipsilateral hemisphere (IH) and the damage area (DA). The percentage of the focal volume to the volume of the ipsilateral hemisphere was calculated according to the following formula: V_{(DA)}/V_{(IH)} = (S_{(DA)}1 + S_{(DA)}2 +...+S_{(DA)}6)/(S_{(IH)}1+S_{(IH)}2 ⁺...+S_{(IH)}6), where S_{(DA)} and S_{(IH)} are the DA and IH areas for each slice (Roda J.M. et al., 1995).

For the purpose of evaluating dynamics of violations in behavior and condition of the animals a complex of methods used in neuropsychopharmacology was applied (T.A. Voronina, 2000). Rats' training and memory were studied on a model of the conditioned reflex of passive avoidance (CRPA, a unit "Passive avoidance" from Lafayette Instrument Co., USA). Animals were trained for CRPA in 24 hours after the operation, and reflex repetition was conducted in a day after training for 3 minutes (180 seconds).

Neurologic status of the animals was determined on the McGrow's Stroke-index scale for Mongolian gerbils (McGrow, 1977) in the modification by I.V. Gannushkina (1996). Evaluation was carried out by scores. If several symptoms of neurological deficit were present in animals, status severity was determined as a sum of corresponding scores. Numbers of rats with light (Stroke-index from 0.5 to 2.5) and severe (Stroke-index from 3 to 10) neurologic symptomatology were counted.

The studies were carried out on male rats of the Vistar line (250-300 grams) held in a vivarium with free access to meals and water and at 12-hour mode of changing day and night.

In the course of the study the animals were randomly distributed over 11 groups. Phenotropil in doses of 100 mg/Kg, 200 mg/Kg and 300 mg/Kg was injected intraperitoneally in three groups of animals 60 minutes prior to the operation and in three groups of animals in 5 minutes after the operation. A saline was injected in falsely operated animals. Then the solutions were injected once on the 2nd and the 3rd days. The solutions were injected strictly at the same time of the days ± 3 minutes.

An analysis of the neurologic status of the animals, which was registered on the McGrow Stroke-index scale, showed that in all the operated animals light impairment of the neurologic status were observed in forms of tremor, lowering of irritability, slowness of motions, unilateral semiptosis. The development dynamics for impairment of the neurologic status for 72 hours after the operation is shown in Table 8.

It should be noted that already on the first day tremor and slowness of motions were less frequently observed in the animals which received Phenotropil prior to the operation (see Table 8). If flabbiness, slowness of motions, tremor in the group of control animals with occlusion of the medial cerebral artery, which did not receive the agent, were identified on the 2nd and the 3rd days, then the animals, which received Phenotropil in the post-operation period, exhibited more rapid positive dynamics: the number of animals with tremor and slowness of motions significantly lowered on the 2nd day. Pronouncement and growth of left-side semiptosis in animals may be evidence of a post-operation edema.

In the course of studying processes of training and memory on a model of the CRPA the findings shown in Table 9 were obtained. Pronounced memory impairment occurred in animals with OMCA. On the background of Phenotropil the latent time of entering the dark section of a chamber significantly increased for all the groups of animals with OMCA compared to that for Group 5. When Phenotropil was used for prophylaxis 60 minutes to OMCA, these indices had no reliable differences with Groups 1 and 2, which indicated high neurotropic effect of the agent.

In 72 hours after the operation the area of damage in control animals (Group 5) was 11.5±0.98% of the ipsilateral hemisphere volume (the calculation formula is given above). The following figures were recorded for animals, which received Phenotropil in doses of 100 mg/Kg, 200 mg/Kg, 300 mg/Kg 60 minutes prior to the operation: 7.4±0.72% (Group 6); 6.52±0.58% (Group 7); 6.03+0.75% (Group 8). Post-operation injection of Phenotropil (in 5 minutes after occlusion of the medial cerebral artery) in doses of 100 mg/Kg, 200 mg/Kg, 300 mg/Kg gave the following results: 11+0.87% (Group 9); 10.79+0.97% (Group 10); 9.5 +0.94% (Group 11) (see Table 10).

The work findings show that Phenotropil preserves the memory functions, improves the neurological status of animals in post-operation periods, which was evidenced by an increase in the latent time of first entering into the dark section of a chamber at the CRPA, by more active behavior of animals, less tremor frequency compared to that in control Group 5. Prophylactic injections of the agent (60 minutes prior to the operation) resulted in a significant reduction in the focal damage area: at 100 mg/Kg - by 36%; 200 mg/Kg - by 43%; 300 mg/Kg - by 48% compared to results in the control group of animals. Phenotropil injection in doses of 100 mg/Kg, 200 mg/Kg and 300 mg/Kg in 5 minutes after the operation resulted in reductions in the brain damage area by 4%, 6% and 17%, respectively.

It was shown that the dependence "dose-effect" at ischemic stroke was most important in the period of acute manifestation of stroke, when higher doses were required. Phenotropil exhibits its uppermost efficiency at a dose of 300 mg/Kg and especially at prophylactic use.

### Example 3. Evaluation of anti-stroke activity of Phenotropil at course injections for 10 days.

A study was conducted according to the methodology described in Example 2 in two groups of animals (20 rats in each group). Phenotropil (300 mg/Kg) and a saline in equivalent volumes were injected intraperitoneally for 10 days (at the same time of the day ± 3 minutes), starting from the 1st day in 5 minutes after the operation. Rats for experimental studies were selected so as they had similar body weight (270 grams ± 10 grams).

Ipsilateral hemisphere volumes were determined on the 3rd and the 10th days of the experiment, as well as the pathological-morphologic status of the hemisphere and the brain damage area were studied.

Scarring of the damage area was noted in both groups on the 10th day. Shrinkage of the hemisphere (contraction to the damage area) was observed in the control group unlike the group of rats which received Phenotropil, and no shrinkage of the hemisphere was identified in the group on Phenotropil on the 10th day. The ipsilateral hemisphere volume in the first group of animals was reduced by 10% at the average (see Table 11). No reliable changes in the hemisphere volume were identified in the second group, which was evidence of a therapeutic effect even after short-term course use of Phenotropil.

The studies conducted show that Phenotropil exhibits pronounced universal neurotropic and cerebrovascular activity, having anti-stroke action in the conditions of experimental hemorrhagic and ischemic strokes as forms of CNS pathology.

On the background of using Phenotropil in an acute period of CNS disorders at cerebrovascular pathology of various etiology the functional memory norm, behavior reactions, motion activity and muscular tonus are reconstructed, and pronounced neurologic deficits are removed with full restoration of psychoneurologic status after course injections of the agent at sufficiently early studies of the disease.

At a hemorrhagic stroke injection of a practically minimum therapeutic dose of the agent (100 mg/Kg) prevented death of animals in 100% of cases. At the same time, 57% of the rats died in the control group.

At an ischemic stroke Phenotropil, which was injected 60 minutes before the operation and once a day for three days, reduced a brain damage area by 42% at the average (48% when using a dose of 300 mg/Kg), which evidenced its efficiency and prospects of use for the purpose of prophylaxis and treatment of cerebrovascular disorders and development of CNS diseases. Injections of Phenotropil in doses of 100, 200 and 300 mg/Kg in 5 minutes after operations and according to the 3-day scheme exhibited a less pronounced effect (17% at a dose of 300 mg/Kg) for this index during a period of stroke acute manifestation, but prolongation of the treatment course up to 10 days contributed to prevention of destructive changes in the brain.

The efficiency of Phenotropil as found for strokes of various etiology goes beyond its separate pharmacological properties, which were described earlier, as described in RU 2 050 851 C1; Selyanina and Shutov "Experience in applying phenotropyl in patients with initial manifestations of insufficient blood circulation in the brain" Nervous Diseases, no 4, 2005; Gerasimova: "Clinical and immunological effects of phenotropyl on the effects of cerebral stroke", Journal of Neurology and Psychiatry, no 5, 2005; Atrapkina and Voronina. The spectrum of pharmacological effects of phenotropyl" Pharmatetra, No. 13, 2005 and shows that Phenotropil is a parent agent for medicines of a new generation, which exhibit universal neurotropic activity and can ensure commensurable (neuromodulator) restructuring of the CNS functional state, which, in its turn, leads to various multicomponent effects, including cerebrovascular-neuroprotective properties, depending on some or other CNS disorders or diseases.

### Bibliography

1.N.V. Vereschagin, Yu.Ya. Varakin. Stroke registers in Russia: results and methodological aspects of the problem // Journ. of neurol. and psychiatr., named after S.S. Korsakov (Supplement "Stroke"). - M., 2001. No. 1, p. 34-40.
2.Internal diseases, ed. by T.R. Harrison et al. M.: Meditsina, 1997. V. 10, 494 pages.
3.T.A. Voronina, R.U. Ostrovskaya. Methodical Guidance on studying nootropic activity of pharmacological substances // In the book: Manual on experimental (preclinical) studies of new pharmacological substances. M., 2000. p. 153-158.
4.T.A. Voronina, S.B. Seredenin. Methodical Guidance on studying tranquilizing (anxiolytic) action of pharmacological substances // In the book: Manual on experimental (preclinical) studies of new pharmacological substances. M., 2000. p. 126-130.
5.I.V. Gannushkina. Pathophysiological mechanisms of cerebral blood flow and new directions in their prophylaxis and treatment // J. of neuropathol. and psychiatr. M. 1996. No. 1, p. 14-18.
6.I.V. Gannushkina. Functional angioarchitecture of brain. M., Meditsina, 1977, p. 224.
7.Ye.I. Gusev. The problem of stroke in Russia. // Journ. of neurol. and psychiatr., named after S.S. Korsakov (Supplement "Stroke"). M., 2003. No. 9, p. 3-7.
8.Ye.I. Gusev, V.I. Skvortsova. Cerebral ischemia. M., Meditsina, 2001.
9.Ye.I. Gusev, V.I. Skvortsova, L.V. Stakhovskaya. Epidemiology of stroke in Russia. // Journ. of neurol. and psychiatr., named after S.S. Korsakov (Supplement "Stroke"). M., 2003. No. 8, p. 4-9.
10. A.N. Makarenko, N.S. Kositsin, S.V. Karpenko, V.A. Mishina. Inventor's Certificate No. 1767518 of 03.11.1990.
11. Manual on experimental (preclinical) studies of new pharmacological substances. M., 2000, p. 159-161.
12. Bederson J.B., Pitts L.H., Germano S.M., Nishimura M.C., Davis R.L,, Bartkowski H.M. Evaluation of 2,3,5-triphenyltetrazolium chloride as a stain for detection and quantification of experimental cerebral infarction in rat//Stroke.-1986.-V.17.-P. 1304-1308.
13. Chen S.T., Hsu C.Y., Hogan E.L., Maricq H., Balentine J.D. A model of focal ischemic stroke in the rat reproducible extensive cortical infarction // Stroke. - 1989. - V.17, No. 4. - P. 738-743.
14. Jackowski A., Crockard A., Burnstock G., Ross Russell R., Kristek F. The time course of intracranial pathophysiological changes following experimental subarachnoid haemorrage in the rat // Journal of cerebral blood flow and metabolism. - 1990. -V. 10. -P. 835-849.
15. Roda J.M., Carceller F., Diez- Tejedor E., Avendano C. Reduction of infarct size by intra-arterial nimodipine administered at reperfusion in a rat model of partially reversible brain focal ischemia // Stroke. - 1995. - V.26, No. 10, p. 1888-1892.
16. Smith S., Hodges H., Sowinski P. Long-Term beneficial effects of BW619C89 on neurological deficit, cognitive deficit and brain damage after middle cerebral artery occlusion in the rat // Neuroscience. - 1997. - V. 77, No. 4, p. 1123-1135.

**Table 1. Influence of Phenotropil (100 mg/Kg, internally) on neurologic deficit in rats on the 1st day of hemorrhagic stroke (on McGrow's scale)**

| **Neurologic symptoms** | **Number of animals with various neurologic symptomatology, %** | | |
|---|---|---|---|
| | **1 day after operation** | | |
| | **Groups of animals** | | |
| | **Falsely operated + saline** | **Stroke + saline** | **Stroke + Phenotropil** |
| Flabbiness, slowness of motions | 40 | 100 | 50 |
| Weakness of limbs | 30 | 90 | 40* |
| Manege movements | 0 | 40 | 0* |
| Paresis of 1-4 limbs | 0 | 30 | 30 |
| Paralysis of 1-4 limbs | 0 | 30 | 0* |

| | | | |
|---|---|---|---|
| * - reliability of differences from stroke rats at p≤0.05 (χ²) | | | |

**Table 2. Influence of Phenotropil on muscular tonus of animals in the horizontal bar test after hemorrhagic stroke**

| **Group of animals** | **Number of animals unable to pull up on a horizontal bar, in a. u. and %** | | | | | | | |
|---|---|---|---|---|---|---|---|---|
| | **1st day** | | **3rd day** | | **7th day** | | **14th day** | |
| | **a. u.** | **%** | **a. u.** | **%** | **a. u.** | **%** | **a. u.** | **%** |
| Intact (w/o operation) | 0/10 | 0 | 0/10 | 0 | 0/10 | 0 | 0/10 | 0 |
| Falsely operated | 1/10 | 10 | 2/10 | 20 | 1/10 | 10 | 0/9 | 0 |
| Stroke | 10/23 | 43* | 9/18 | 50* | 5/13 | 38* | 4/11 | 36* |
| Stroke + Phenotropil | 5/12 | 42 | 4/12 | 33 | 3/12 | 25 | 2/12 | 16** |

| | | | | | | | | |
|---|---|---|---|---|---|---|---|---|
| a.u. = absolute units * - reliability of differences from falsely operated rats at p≤0.05 <χ²) ** - reliability of differences from stroke rats at p≤0.05 (χ²) | | | | | | | | |

**Table 3. Influence of Phenotropil on movement coordination in animals in the Rotarod test after hemorrhagic stroke**

| **Group of animals** | **Number of animals unable to hold up on a rotating rod (3 rpm) for 2 minutes, in a. u. and %** | | | | | | | |
|---|---|---|---|---|---|---|---|---|
| | **1st day** | | **3rd day** | | **7th day** | | **14th day** | |
| | **a. u.** | **%** | **a. u.** | **%** | **a. u.** | **%** | **a. u.** | **%** |
| Intact (w/o operation) | 0/10 | 0 | 0/10 | 0 | 0/10 | 0 | 0/10 | 0 |
| Falsely operated | 2/10 | 20 | 3/10 | 30 | 1/10 | 10 | 1/9 | 10 |
| Stroke | 11/23 | 48* | 9/18 | 50 | 5/13 | 38* | 5/11 | 45* |
| Stroke + Phenotropil | 5/12 | 42 | 4/12 | 33 | 0/12 | 0** | 0/12 | 0** |

| | | | | | | | | |
|---|---|---|---|---|---|---|---|---|
| a.u. = absolute units * - reliability of differences from falsely operated rats at p≤0.05 (χ²) ** - reliability of differences from HS rats at p≤0.05 (χ²) | | | | | | | | |

**Table 4. Influence of Phenotropil on performing the burrow reflex.**

| **Groups of animals** | **Latent time of entering into a dark chamber during training in the burrow reflex** |
|---|---|
| | **1 day after operation** |
| Intact animals (w/o operation) | 12.8±1.2 |
| Falsely operated animals | 13.03±0.9 |
| Stroke animals | 35.2±10.1 |
| Phenotropil | 30.6±8.8 |

**Table 5a. Influence of Phenotropil on repetition of the CRPA in rats with intracerebral post-traumatic hematoma.**

| | **Repetition of the CRPA in:** | | | |
|---|---|---|---|---|
| **Groups of animals** | **24 hours after training** | | **3 days** | |
| | **Latent time of entering into a dark chamber, sec** | **Number of rats which did not entered a dark chamber, %** | **Latent time of entering into a dark chamber, sec** | **Number of rats which did not entered a dark chamber, %** |
| Intact (w/o operation) | 156.0±24.0 | 80 | 152.5±14.3 | 80 |
| Falsely operated | 143.7±16.3 | 70 | 137.6±17.4 | 65 |
| Stroke | 68.3±26.4 | 25* | 71.4±39.3* | 28 |
| Stroke + Phenotropil | 101.1±24.2** | 40** | 98.3±18.7 | 40** |

| | | | | |
|---|---|---|---|---|
| * - reliability of differences from falsely operated rats at p≤0.05 (χ²) ** - reliability of differences from HS rats at p≤0.05 (χ²) | | | | |

**Table 5b.**

| | **Repetition of the CRPA in:** | | | |
|---|---|---|---|---|
| **Groups of animals** | **7 days** | | **14 days** | |
| | **Latent time of entering into a dark chamber, sec** | **Number of rats which did not entered a dark chamber, %** | **Latent time of entering into a dark chamber, sec** | **Number of rats which did not entered a dark chamber, %** |
| Intact (w/o operation) | 143.1±16.5 | 75 | 133.6±25.1 | 60 |
| Falsely operated | 124.3±22.0 | 60 | 114.2±20.6 | 55 |
| Stroke | 28.0±2.9* | 16* | 23.7±11.2* | 9* |
| Stroke + Phenotropil | 75.2±24.1** | 30** | 83.2±26.7** | 40** |

| | | | | |
|---|---|---|---|---|
| * - reliability of differences from falsely operated rats at p≤0.05 (Student's t-test; χ²) ** - reliability of differences from HS rats at p≤0.05 (Student's t-test; χ²) | | | | |

**Table 6. Influence of Phenotropil on orientation-and-exploratory behavior and motion activity of animals in the conditions of the open-field methodology after hemorrhagic stroke**

| **Groups of animals** | **Horizontal motion activity** | **Vertical motion activity** | **Exploration of holes** | **Total index** |
|---|---|---|---|---|
| **1st day after operation** | | | | |
| Intact | 15.6±2.3 | 5.6±1.4 | 3.3±0.9 | 24.5±5.4 |
| Falsely operated | 14.7±1.9 | 2.9±0.6 | 3.2±0.7 | 20.8±2.6 |
| HS + saline | 7.3±1.1* | 2.7±0.8 | 1.8±0.3 | 11.8±2.0* |
| HS + Phenotropil | 8.8±3.5 | 4.4±2.1 | 1.3±0.5 | 14.5±5.5 |
| **3rd day after operation** | | | | |
| Intact | 14.0±1.2 | 5.1±1.1 | 3.2±0.8 | 22.3±2.5 |
| Falsely operated | 13.7±2.3 | 3.2±0.8 | 3.1±0.5 | 20.0±2.3 |
| HS + saline | 6.2±1.2* | 2.7±0.4 | 1.7±0.5 | 10.6±1.5 |
| HS + Pheno- tropil | 15.2±5.4 | 4.5±1.5 | 1.9±0.7 | 21.6±6.1** |
| **7th day after operation** | | | | |
| Intact | 13.6±2.3 | 5.3±1.4 | 4.1±1.2 | 23.0±5.7 |
| Falsely operated | 13.4±2.1 | 4.1±2.8 | 2.9±0.6 | 20.4±3.5 |
| HS + saline | 7.8±1.9 | 3.2±1.3 | 2.4±0.5 | 13.4±2.9 |
| HS + Phenotropil | 20.9±4.1** | 10.9±3.2 | 4.6±1.3 | 36.4±5.6 |
| **14th day after operation** | | | | |
| Intact | 12.4±2.5 | 5.6±1.3 | 3.5±1.2 | 21.5±5.9 |
| Falsely operated | 13.4±2.2 | 6.2±1.4 | 3.3±0.6 | 22.9±3.5 |
| HS + saline | 8.1±2.4* | 3.7±1.4 | 3.2±1.3 | 15.0±3.7* |
| HS + Phenotropil | 12.2±2.8 | 6.9±1.9 | 3.0±0.7 | 22.1±3.5** |

| | | | | |
|---|---|---|---|---|
| * - reliability of differences from falsely operated rats at p≤0.05 (Student's t-test; χ²) ** - reliability of differences from HS rats at p≤0.05 (Student's t-test; χ²) | | | | |

**Table 7. Influence of Phenotropil on survivability of animals after HS.**

| **Group of animals** | **Number of animals died within 14 days after hemorrhagic stroke** | | | | | | | |
|---|---|---|---|---|---|---|---|---|
| | **1st day** | | **3rd day** | | **7th day** | | **14th day** | |
| | **a. u.** | **%** | **a. u.** | **%** | **a. u.** | **%** | **a. u.** | **%** |
| Falsely operated | 0/10 | 0 | 0/10 | 0 | 0/10 | 0 | 1/10 | 10 |
| HS + saline | 7/30 | 23* | 5/23 | 22* | 5/18 | 28* | 2/13 | 15* |
| Stroke + Phenotropil | 0/12 | 0** | 0/12 | 0** | 0/12 | 0** | 0/12 | 0** |

| | | | | | | | | |
|---|---|---|---|---|---|---|---|---|
| * - reliability of differences from falsely operated rats at p≤0.05 (χ²) ** - reliability of differences from HS rats at p≤0.05 (χ²) | | | | | | | | |

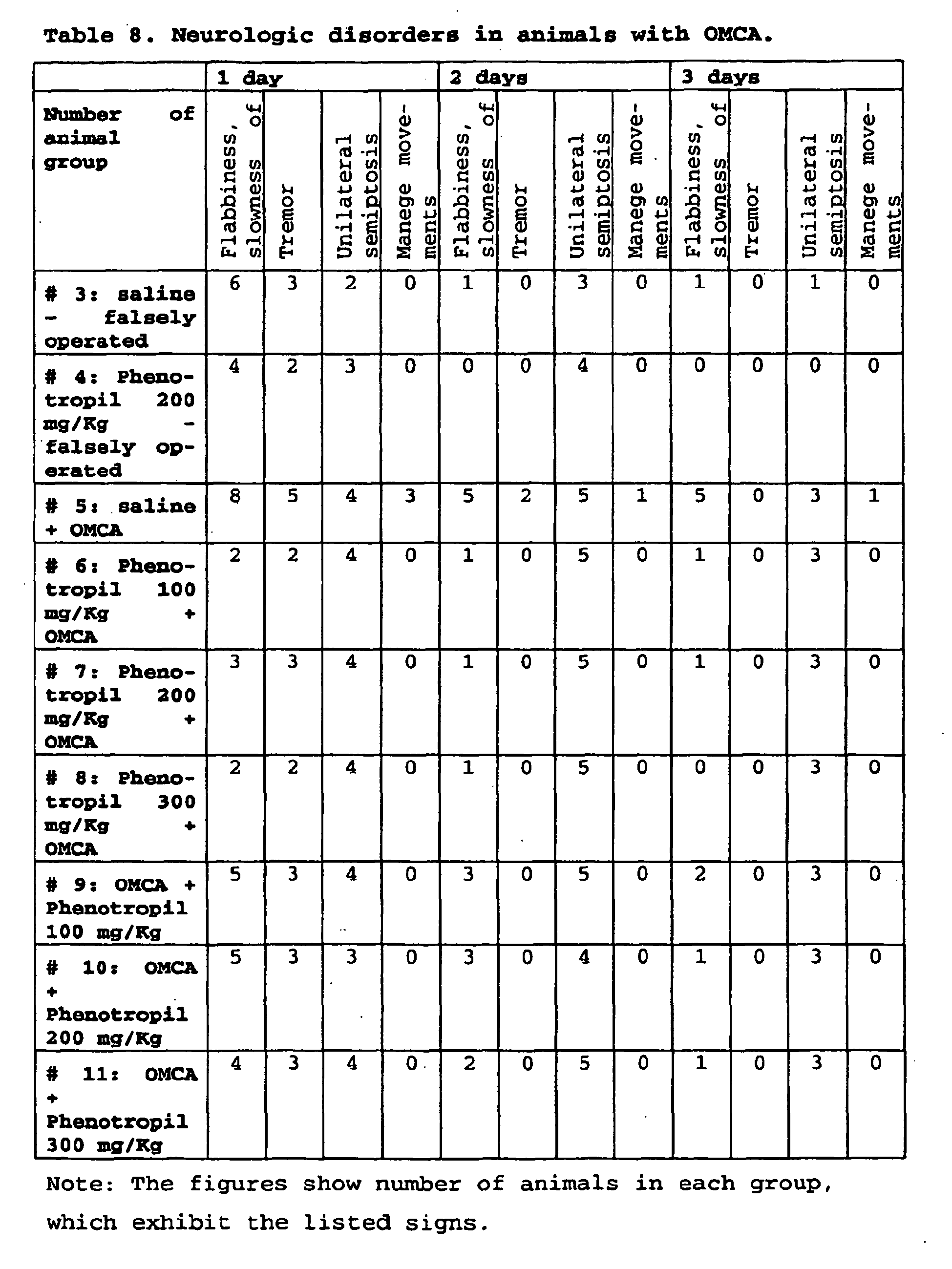

**Table 9. Influence of Phenotropil on training (CRPA) of rats during an ischemic stroke acute period (OMCA) in 24 hours after operation and during testing repetition of CRPA maintenance in 24 hours after training**

| **No.** | **Groups of animals** | **Latent time of first entering into a dark chamber section** |
|---|---|---|
| 1 | Control: saline | 101.2±19.0 |
| 2 | Control: Phenotropil w/o OMCA | 105.9±25.7 |
| 3 | Saline, falsely operated | 69.7±20.7 |
| 4 | Phenotropil 200 mg/Kg, falsely operated | 95.9±24.9 |
| 5 | Saline + OMCA | 48.5±17.5* |
| 6 | Phenotropil 100 mg/Kg + OMCA | 93.6±22.3** |
| 7 | Phenotropil 200 mg/Kg + OMCA | 104.6±18.8** |
| 8 | Phenotropil 300 mg/Kg + OMCA | 90.6±22.6** |
| 9 | OMCA + Phenotropil 100 mg/Kg | 83.3±26.1** |
| 10 | OMCA + Phenotropil 200 mg/Kg | 84.5±19.6** |
| 11 | OMCA + Phenotropil 300 mg/Kg | 67.6±18.4** |

| | | |
|---|---|---|
| * - reliability of differences in relation to Group 1 at p≤0.05 ** - reliability of differences in relation to Group 5 at p<0.05 | | |

**Table 10. Size of brain damage area at OMCA.**

| **Groups of animals** | **Volume of ipsilateral hemisphere (IH), mm³** | **Volume of damage area (DA), mm³** | **DA/IH relation, %** |
|---|---|---|---|
| Group 5 | 765.6±3.77 | 88±7.57 | 11.5±0.98 |
| Group 6 | 786.6±4.74 | 58.2±5.71 | 7.39±0.72* |
| Group 7 | 739.77±4.8 | 49.2±6.13 | 6.52±0.58* |
| Group 8 | 797±4.33 | 48.1±5.99 | 6.03±0.75 |
| Group 9 | 787.9±5.24 | 86.5±6.53 | 11±0.87 |
| Group 10 | 794.8±4.27 | 85.9±7.78 | 10.8±0.97 |
| Group 11 | 801.2±3.63 | 76.2±7.59 | 9.5±0.94 |

| | | | |
|---|---|---|---|
| * p<0.01 in comparison to Group 5 (control animals with OMCA w/o the agent). | | | |

**Table 11. Change in volume of the ipsilateral hemisphere at OMCA.**

| **No.** | **Groups of animals** | **Volume of ipsilateral hemisphere, mm³, on the 3rd day** | **Volume of ipsilateral hemisphere, mm³, on the 10th day** |
|---|---|---|---|
| 1 | OMCA + saline | 770.74±72 | 693.53±4.12* |
| | OMCA + Phenotropil 300 mg/Kg | 772.4±4.5 | 773.17±3.83 |

| | | | |
|---|---|---|---|
| * - reliability of differences in relation to control on the 3rd day for Group 1 at p<0.01. | | | |

## Claims

1. The use of N-carbomoyl-methyl-4-phenyl-2-pyrrolidon for the manufacture of a medicament for the prophylaxis and treatment of hemorrhagic stroke and acute phase of ischemic stroke.

2. N-carbomoyl-methyl-4-phenyl-2-pyrrolidon for use in the prophylaxis and treatment of hemorrhagic stroke and acute phase of ischemic stroke.

## Patentansprüche

1. Verwendung von N-carbomoyl-methyl-4-phenyl-2-pyrrolidon für die Herstellung eines Medikaments für die Prophylaxe und die Behandlung von hämorrhagischen Schlaganfall und die Akutphase von ischämischen Schlaganfall.

2. N-carbomoyl-methyl-4-phenyl-2-pyrrolidon zur Verwendung in der Prophylaxe und Behandlung von hämorrhagischen Schlaganfall und der Akutphase von ischämischen Schlaganfall.

## Revendications

1. Utilisation du N-carbomoyl-methyl-4-phenyl-2-pyrrolidon pour la fabrication d'un médicament destiné à la prophylaxie et au traitement d'un accident hémorragique ou de la phase aiguë d'un accident ischémique.

2. N-carbomoyl-methyl-4-phenyl-2-pyrrolidon pour être utilisé dans la prophylaxie et le traitement d'un accident hémorragique et de la phase aiguë d'un accident ischémique.
